# FASCICULE DE BREVET EUROPEEN

(11) **EP 1 981 549 B1**
(45) Date de publication et mention de la délivrance du brevet: **13.05.2009**
(21) Numéro de dépôt: 06847095.4
(22) Date de dépôt: 19.12.2006
(51) Int. Cl.: A61L 2/00

(54) **PROCEDE D'INACTIVATION VIRALE PAR CHAUFFAGE A SEC**
VERFAHREN ZUR INAKTIVIERUNG VON VIREN DURCH TROCKENE HITZE
METHOD OF VIRAL INACTIVATION BY DRY HEATING

(30) Priorité: 19.12.2005 FR 0512875
(43) Date de publication de la demande: 22.10.2008
(73) Titulaire: Laboratoire Francais du Fractionnement et des Biotechnologies Societe Anonyme, 91940 Les Ulis (FR)
(72) Inventeur: BARDAT, Annie, F-91470 Limours (FR)
(74) Mandataire: Vieillefosse, Jean-Claude
(86) Numéro de dépôt international: PCT/FR2006/002817
(87) Numéro de publication internationale: WO 2007/071845

(56) Documents cités:
- EP-A- 0 094 611
- EP-A- 0 844 005
- US-A- 5 831 027
- HART H F ET AL: "EFFECT OF TERMINAL (DRY) HEAT TREATMENT ON NON-ENVELOPED VIRUSES IN COAGULATION FACTOR CONCENTRATES" VOX SANGUINIS, S. KARGER AG, BASEL, CH, vol. 67, 1994, pages 345-350, XP000667216 ISSN: 0042-9007 cité dans la demande

## Description

### DOMAINE DE L'INVENTION

Toute matière biologique solide présente un risque de contamination virale et celle-ci, ou ses produits dérivés, ou des produits issus de procédés dans lesquels elle interviendrait, doivent être soumis à des procédés d'inactivation virale en vue d'être utilisés en thérapie ou en prophylaxie.

On utilise dans le domaine thérapeutique ou prophylactique des principes actifs issus de sources biologiques ou des principes actifs susceptibles d'être contaminés par une source biologique au cours de leur procédé de fabrication.

Ces principes actifs peuvent être des protéines, peptides, polypeptides, anticorps, éventuellement substitués par des groupes lipidiques ou glucidiques, des acides nucléiques, ADN, ARN, des polysaccharides, des bactéries, des particules virales et autres.

La source biologique dont ils sont issus ou susceptible de contaminer leur procédé d'obtention peut être n'importe quel tissu humain ou animal, le sang, le plasma, les os, n'importe quel tissu végétal, n'importe quel micro-organisme, un milieu de culture cellulaire, de culture de virus, de bactéries, de levures, de moisissures, de champignons.

Par conséquent, il est courant d'inclure dans les étapes d'extraction des principes actifs produits à partir de telles sources biologiques, des étapes de réduction ou inactivation virale.

On entend par produit biologique au sens de la présente invention un produit comprenant un principe actif produit à partir d'une source biologique et d'autres composés ou excipients provenant du procédé d'obtention dudit principe actif.

Des méthodes d'inactivation virale par traitement avec des produits chimiques et/ou la chaleur sont connues de l'art antérieur. La grande majorité de celles-ci provient du domaine de la transfusion sanguine dans lequel l'efficacité d'une inactivation virale est cruciale puisqu'il faut tenter de s'affranchir de la contamination éventuelle résultant de produits issus d'un donneur.

Le chauffage a été préconisé dès le début de la reconnaissance de l'origine virale du VIH pour inactiver celui-ci, en particulier dans le sang, le plasma, les produits biologiques issus du sang ou du plasma. Le chauffage à sec c'est-à-dire le chauffage à une température T pendant un temps t d'un produit sec a été préconisé, par exemple pour les concentrés de facteur de la coagulation lyophilisés ou cryodesséchés qui n'ont pas été chauffés sous forme liquide. Par exemple, le facteur VIII de la coagulation sanguine, extrait du plasma humain, a été chauffé à 60°C pendant 72 à 96 h à l'état lyophilisé pour sécuriser ce principe actif biologique destiné à traiter les hémophiles. Cependant, l'inconstance des réductions de titre viral a fait abandonner ce procédé car plusieurs cas de contamination par le VIH chez les hémophiles ont été recensés malgré ce chauffage.

Il a donc été proposé de soumettre ces produits à des conditions de chauffage dites « sévères », c'est-à-dire une température de 80°C pendant. 72h à l'état sec.

Ce procédé d'inactivation virale a alors été validé pour le VIH (virus enveloppé) suite aux résultats cliniques obtenus pour.un facteur VIII traité dans ces conditions (L. Winkelman et al., « Severe Heat Treatment of Lyophilised Coagulation Factors », Curr Stud Hematol Blood Transfus, 1989, n°56, p55-69).

Le traitement des protéines purifiées avec un mélange de solvant et de détergent est également largement utilisé pour prévenir la transmission des virus enveloppés par des protéines dérivées de sources biologiques (Piet et al., Transfusion, 30 :592-98, 1990). Ce traitement est efficace contre les virus présentant une enveloppe lipidique, beaucoup moins contre ceux qui ne présentent pas une telle structure. Récemment, la transmission d'au moins deux virus non-enveloppés par l'utilisation d'un produit biologique traité par solvant/détergent a été décrite. Le virus de l'hépatite A, virus à ARN non-enveloppé, a été transmis à des patients utilisant un Facteur VIII qui avait été traité par solvant/détergent (Purcell et al., Vox Sang, 67 :2-7, 1994). Le Facteur VIII a également été impliqué dans la transmission d'un parvovirus non-enveloppé, B19 (Lefrere et al., Lancet, 343 :211-12, 1994).

Le traitement des protéines purifiées par chauffage a été préconisé pour étendre le spectre d'inactivation virale aux virus non-enveloppés. Cependant, l'inactivation par chauffage de virus non-enveloppés est généralement plus difficile que celle des virus enveloppés et requiert souvent un traitement plus long et/ou des températures plus élevées pour assurer une inactivation satisfaisante. B19 a été transmis à des patients par un Facteur VIII qui avait été chauffé à sec à 100°C pendant 30 min (Santagostino et al., Lancet 343 :798, 1994). Il est donc clairement important dé déterminer comment améliorer les méthodes d'inactivation virale pour maintenir ou améliorer la sécurité des produits biologiques.

### ART ANTERIEUR

De nombreux auteurs ont cherché à observer les paramètres majeurs qui influent sur l'inactivation virale par chauffage à sec. Leur but est de déterminer un paramètre physico-chimique qui permettrait de prédire si un traitement donné est adapté à la matière solide à traiter ou non c'est-à-dire s'il permet une inactivation virale satisfaisante tout en conservant une stabilité satisfaisante du produit. De plus, il serait extrêmement intéressant que ce paramètre soit modulable et qu'en fonction de cette modulation on puisse favoriser l'inactivation virale ou la stabilité du produit.

On définit le facteur de réduction virale d'un procédé d'inactivation virale comme le facteur de réduction du titre viral c'est-à-dire le log₁₀ du rapport du titre viral avant l'étape d'inactivation au titre viral après l'étape d'inactivation.

On définit le taux d'humidité comme la quantité d'eau en poids pour 100g de produit. C'est pourquoi il est exprimé en pourcentage en poids. La méthode classique de mesure consiste à déterminer la perte en poids du produit après chauffage à une température supérieure à 100°C jusqu'à ce que le poids du produit soit constant.

Willkommen et al. (Paul Ehrlich Institute) ont montré que pour les lyophilisats à faible taux d'humidité (< 0,8%), les facteurs de réduction du virus de l'hépatite A (VHA) obtenus par chauffage à 80°C pendant 72 h vont de 0 à 0,4 log₁₀ alors que pour les lyophilisats à fort taux d'humidité (> 0,8%), les facteurs de réduction du virus de l'hépatite A obtenus dans les mêmes conditions sont supérieurs ou égaux à 4,3 log₁₀.

Bunch et al. (Alpha Therapeutic Corporation) ont montré que deux taux d'humidité différents (0,4 et 1,4%) d'un facteur VIII recombinant n'avaient aucune influence sur le facteur de réduction du virus de l'hépatite A (≥ 6,9 log₁₀) par chauffage à 80°C pendant 72h.

Roberts PL et al. (Biologicals. 2000 Sep; 28(3): 185-8., "Comparison of the inactivation of canine and bovine parvovirus by freeze-drying and dry-heat treatment in two high purity factor VIII concentrates") ont montré l'influence de la formulation du produit biologique et de la résistance du virus par inactivation virale de 2 parvovirus bovin et canin par chauffage à 80°C pendant 72h d'un concentré de Facteur VIII dans. 2 formulations lyophilisée.

Hart HF et al. (Vox Sang. 1994; 67 (4) :345-50 "Effect of terminal (dry) heat treatment on non-enveloped viruses in coagulation factor concentrates") ont obtenu le même facteur de réduction du virus de l'hépatite A dans des lyophilisats de Facteur VIII par chauffage à 80°C pendant 24h ou 90°C pendant 2h.

Tomokiyo et al. (Vox Sang. 2003 Jan ; 84(1):54-64. "Large-scale production and properties of human plasma-derived activated Factor VII concentrate") ont montré, par inactivation de différents virus : CMV (Cytomégalovirus), VIH (Virus de l'Immunodéficience acquise Humain), BVDV (Virus de la Diarrhée Virale Bovin), Poliovirus, PPV (Parvovirus Porcin) dans des lyophilisats de Facteur VIIa, que l'inactivation virale dans des lyophilisats est possible à 65°C. Un chauffage à 65°C pendant 96h sur des produits dont le taux d'humidité est < 1,7% montre des facteurs de réduction virale > 4 log₁₀ sur l'ensemble des virus sauf le PPV.

La demande de brevet EP 0 844 005 divulgue que l'humidité résiduelle du produit biologique desséché à traiter est l'élément critique dans l'efficacité de l'inactivation virale par un procédé de chauffage à sec à 80°C, pendant 72-77h. Les virus testés sont le VHA, le parvovirus porcin et le pseudorabies virus. Les inventeurs ont montré que l'humidité résiduelle doit être supérieure ou égal à 0,8% pour atteindre un facteur de réduction virale ≥ 4 log₁₀ avec ce procédé. Pour une humidité résiduelle ≤ 0,8%, le facteur de réduction virale est de 0,12 log₁₀ en moyenne.

### PROBLEME TECHNIQUE

Il apparaît, au vu de ces résultats très dispersés, qu'aucun paramètre n'a été défini dont la mesure permettrait de déterminer de manière sûre les variables opératoires caractéristiques d'un procédé d'inactivation virale par chauffage à sec à utiliser en fonction du produit biologique à traiter.

Il semble toutefois qu'il existe un certain consensus entre les auteurs sur le fait que le taux d'humidité du produit à traiter joue un rôle très important sans toutefois que ceux-ci soient d'accord sur un taux d'humidité résiduelle en tant que valeur seuil pour obtenir une inactivation virale satisfaisante. En effet, il suffit parfois que cette valeur baisse de quelques dixièmes pour que l'inactivation soit incomplète.

Cependant, contrairement à ce que pourraient laisser penser certains auteurs, le Demandeur a montré- qu'une inactivation virale est possible dans des lyophilisats d'humidité résiduelle faible. Un fibrinogène humain cryodesséché d'humidité résiduelle égale à 0,1% a ainsi été chauffé à sec à 77°C pendant 72 h. Les facteurs de réduction des virus de l'hépatite A (VHA), du virus de l'immunodéficience humain (VIH), du virus de la diarrhée virale bovine (BVDV) et du parvovirus porcin (PPV) sont présentés dans le Tableau 1.

**Tableau 1 :**

| Virus | Facteur de réduction |
|---|---|
| VHA | 4,10 ± 0,3 |
| | 3,75 ± 0,26 |
| VIH | 4,53 ± 0,36 |
| | 4,62 ± 0,30 |
| | 4,88 ± 0,28 |
| BVDV | 5,96 ± 0,40 |
| | 5,21 ± 0,38 |
| PPV | 2,97 ± 0,43 |
| | 2,88 ± 0,37 |

La dispersion de l'ensemble de ces observations ne permet donc que de tirer la conclusion suivante : l'humidité résiduelle du produit à traiter n'est donc pas le facteur déterminant : des résultats de l'inactivation virale par chauffage à sec mais un facteur important dont dépendait le facteur déterminant.

Le problème est donc de déterminer le paramètre physico-chimique multi-factoriel mesurable donnant une valeur seuil de part et d'autre de laquelle l'inactivation virale sera satisfaisante ou insatisfaisante.

### RESUME DE L'INVENTION

Le Demandeur a identifié, de façon surprenante, que ce paramètre physico-chimique mesurable est la température de transition vitreuse du produit biologique à traiter.

La transition vitreuse est une transition du second ordre c'est-à-dire une transition thermique qui implique un changement de capacité calorifique, mais pas de chaleur latente. Elle est caractéristique des liquides surfondus qui sont refroidis à une température suffisamment basse suffisamment rapidement sans cristalliser et qui deviennent un verre et des.. polymères amorphes ou de la partie amorphe des polymères cristallins qui passent d'un état dur et fragile à un état mou et souple.

La température de transition vitreuse ou Tg est la température à laquelle a lieu la transition vitreuse. Quand un polymère est refroidi en-dessous de cette température il devient dur et fragile, comme le verre, on dit qu'il est à l'état vitreux.

Les caoutchoucs élastomères comme le polyisoprène et le polyisobutylène, sont utilisés au-dessus de leur température de transition vitreuse, c'est-à-dire à l'état caoutchouteux et ils sont mous et flexibles.

La température de transition vitreuse est connue de l'homme du métier comme dépendante de certains paramètres. Dans le cas des polymères, elle dépend de la masse moléculaire, de la structure chimique de la chaîne, de la quantité de plastifiants.

Les plastifiants sont des petites molécules, comme les sels, qui s'insinuent entre les molécules de polymères et leur permettent de mieux glisser entre elles donc facilitent leur mouvement. L'ajout de plastifiant permet donc d'abaisser la température de transition vitreuse.

Au contraire les molécules de poids moléculaire élevé bloquent les mouvements des molécules de polymère entre elles et augmentent la température de transition vitreuse.

Par ailleurs, le Demandeur a montré que la température de transition vitreuse est directement corrélée à l'humidité résiduelle d'un lyophilisat de Facteur Von Willebrand (FvW) donné.

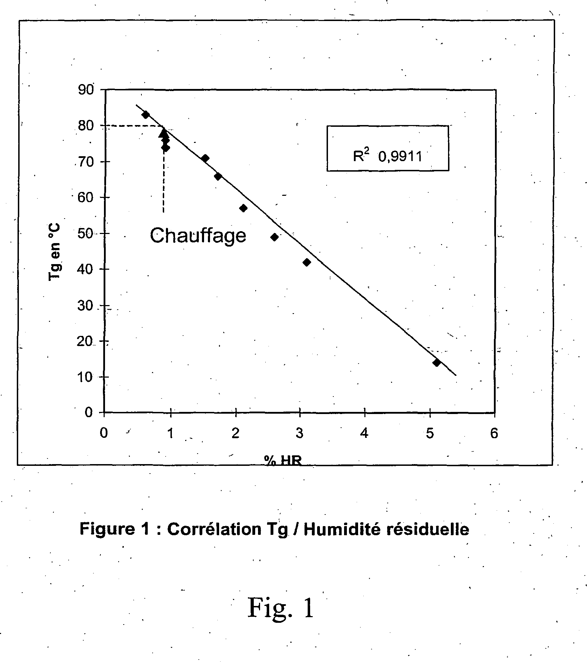

La corrélation entre la température de transition vitreuse du lyophilisat et son humidité résiduelle est présentée sous forme de graphique à la Figure 1.

La température de transition vitreuse d'un produit biologique dépend donc de la nature du principe actif, de la nature des excipients : plastifiants ou non, forme cristalline ou forme amorphe, de la masse moléculaire des excipients et de l'humidité résiduelle du produit biologique.

### DESCRIPTION DETAILLEE DE L'INVENTION

L'invention concerne un procédé d'inactivation virale par chauffage à sec d'un virus présent ou potentiellement présent dans un produit biologique séché, caractérisé par les étapes suivantes :
a) déterminer la température de transition vitreuse Tg du produit biologique séché à traiter puis,
b) chauffer le produit- biologique séché à traiter de l'étape a) à une température de chauffage à sec T supérieure ou égale à la température de transition vitreuse Tg déterminée à l'étape a).

Un produit séché est un produit ayant subi une méthode de dessiccation connue de l'homme du métier comme la lyophilisation, lé séchage sous vide, la pervaporation, l'atomisation.
En particulier, un produit séché est un produit cryodesséché c'est-à-dire un produit tout d'abord congelé dont une partie de l'eau est ensuite sublimée sous vide.

En effet, le Demandeur a observé que le facteur de réduction virale et la cinétique d'inactivation virale sont favorisés lorsque la température de chauffage est supérieure ou égale à Tg.
La valeur de la température de transition vitreuse permet donc de prédire si un procédé d'inactivation sera satisfaisant et de le modifier dans ce sens.

La mesure de la température de transition vitreuse d'un produit biologique séché- consiste à soumettre un échantillon de ce produit à une augmentation progressive et programmée de la température entre -50°C et +100°C et à observer ses changements d'état, dont la transition vitreuse.
On obtient ainsi le thermogramme du produit biologique séché et notamment sa température de transition vitreuse.
Après avoir mesuré la température de transition vitreuse, selon ses connaissances générales dans le domaine de l'inactivation virale par chauffage, l'homme du métier sait juger si pour satisfaire à l'exigence T ≥ Tg :
- Tg est satisfaisante selon le virus concerné pour choisir une température T ≥ Tg
- ou bien si Tg doit être ajustée pour choisir T afin que l'inactivation virale et la stabilité du produit recherchées soient satisfaites.
Par exemple, si l'homme du métier sait que Tg est trop faible pour que le virus en question puisse être inactivé à T de sorte que Tg ≤ T et que le produit reste stable, alors il augmentera Tg pour que T se situe dans une gamme de température qu'il sait pouvoir inactiver ce virus et que l'écart entre T et Tg ne soit pas assez important pour que le produit se dégrade.
Par contre, si l'homme du métier sait que Tg est trop élevée pour que T ≥ Tg et que le produit reste stable alors il diminuera.Tg avant de choisir T.

Le procédé d'inactivation virale par chauffage à sec dans un produit biologique selon l'invention est particulièrement approprié dans le cas d'un virus non-enveloppé.

Ce procédé peut permettre de traiter une composition contenant une ou plusieurs protéines extraits du plasma sanguin en tant que produit biologique séché.
Dans un mode de réalisation particulier, la température de chauffage à sec T est choisie pour permettre l'inactivation d'un virus non-enveloppé.

De manière préférée, la température de transition vitreuse est augmentée par ajout d'excipients de masse moléculaire élevée au produit biologique ou par diminution de l'humidité du produit biologique ou bien elle est diminuée par ajout de sels ou d'excipients de: faible masse moléculaire au produit biologique ou par augmentation de l'humidité du produit biologique.

En particulier, la température de transition vitreuse est mesurée au moyen d'un thermoanalyseur différentiel à balayage. Les changements d'état sont définis par un changement de capacité calorifique mesurée par rapport à un produit inerte qui ne subit pas de transformation dans la plage de température considérée.

On préférera que la température de chauffage T du procédé selon l'invention soit- comprise entre Tg et Tg + 20°C pour conserver une stabilité- du produit satisfaisante. Dans cet intervalle, on pourra soit choisir T de manière à accroître l'écart entre Tg et T dans la limite de Tg +20°C , pour favoriser le facteur de réduction virale et la cinétique d'inactivation virale, soit choisir T de manière à diminuer l'écart entre Tg et T , pour favoriser la stabilité du produit.
De manière particulièrement préférée, la température de chauffage à sec T est choisie pour permettre l'obtention d'un facteur de réduction virale ≥ 3 log₁₀, de préférence ≥ 4 log₁₀.
Dans un mode de réalisation particulier, dans une étape finale, on mesure l'efficacité de l'inactivation virale dans le produit biologique séché traité et, si ladite efficacité est jugée insuffisante, l'inactivation virale de produit biologique séché se poursuit après accroissement d'un écart entre la température de chauffage T et la température de transition vitreuse Tg.
Dans un autre mode de réalisation particulier, dans une étape finale, on évalue la stabilité du produit biologique séché traité et, si ladite stabilité est jugée insuffisant, l'inactivation virale de produit biologique séché se poursuit après une diminution d'un écart entre la température de chauffage T et la température de transition vitreuse Tg.

### Figures

- Figure 1 :: corrélation Tg/HR, Tg = température de transition vitreuse et HR = humidité résiduelle
- Figure 2 :: facteur de réduction de PR772 par chauffage à sec à 62°C en fonction de Tg
- Figure 3 :: facteur de réduction de PR772 par chauffage à sec à 80°C en fonction de Tg
- Figure 4 :: facteur de réduction de PPV par chauffage à sec à 80°C en fonction de Tg
- Figure 5 :: facteur de réduction de -PPV, HAV, BVDV, PR772, Phi174 à T = Tg = 80°C
- Figure 6 :: facteur de réduction de PPV, HAV, BVDV, PR772, Phi174 à T = Tg = 62°C

### Exemples

### Exemple 1 : Inactivation du bactériophage PR772 par chauffage à sec dans des lyophilisats :

Les caractéristiques physiques des lyophilisats sont modifiées afin de faire varier la température de transition vitreuse (Tg).
La température de -transition vitreuse est déterminée par un thermoanalyseur différentiel à balayage. La température du thermoanalyseur différentiel à balayage est calibrée à l'aide de l'indium (Tm 156,6°C) et du n-octadécane (Tm- 28,2°C). Les échantillon subissent des- températures de -50°C à 130°C à la vitesse de 20°C/min. L'azote liquide est utilisé pour mener les expériences à une température inférieure à la température ambiante. La température de transition vitreuse est prise au point médian du changement endothermique dans la chaleur spécifique apparente. Deux mesures sont réalisées et leur moyenne donne la Tg.
Le chauffage est effectué à une température inférieure à Tg, soit à l'état solide vitreux, ou à une température supérieure à Tg d'environ 20°C, soit à l'état viscoélastique (caoutchouteux).
Tous les lyophilisats présentent une teneur en eau inférieure à 1%.
La teneur en eau est déterminée par la méthode de Karl-Fischer, bien connue de l'homme du métier, basée sur la réaction entre l'eau et l'iode.

### Formulation du produit A (pH 7,0 + 0,5) :

- glycine 7,5g/l
- lysine HCl 5,5 g/l
- CaCl2 0,15 g/l
- mannitol 40 g/l
- saccharose 50 g/l
- FVIII 100 UI/ml
Le produit A présente une Tg de 62°C.
Le produit B présente la même formulation que le produit A à laquelle on a ajouté du NaCl. Ceci a permis de diminuer la Tg à environ 40°C (l'humidité résiduelle HR reste inchangée).
C est un concentré de FvW cryodesséché et D est un fibrinogène humain cryodesséché.

### Formulation du produit C (pH 7,0 ± 0,5) :

- citrate trisodique 10mM
- CaCl2 1 mM
- glycine 5 g/l
- arginine HCl 40 g/l
- albumine 10 g/l
- FvW 100 UI/ml

### Formulation du produit D (6,8 < pH < 7,2) :

- fibrinogène 11 à 20 g/l
- chlorhydrate d'arginine 40 g/l
- isoleucine 10 g/l
- glycine 2 g/l
- monochlorhydrate de lysine 2 g/l
- tri-sodium citrate 2 H2O 2,5 g/l

Les produits C et D présentent des Tg respectivement de 80°C et 90°C.
On mesure le facteur de réduction du bactériophage PR772 à 12, 24 et 72h pour un chauffage à 62 et 80°C.
Le titre viral est calculé selon la formule de Spearman Kärber tel que décrit dans the Federal Gazette n°84, 4 mai 1994 et dans Schmidt, N.J. et Emmons, R.W. (1989) dans Diagnostic Procedures for Viral, Rickettsial and Chlamydial Infection, 6ème Edition.
Le facteur de réduction est le résultat du rapport entre le titre viral/ml avant le traitement par chauffage à sec et le titre viral/ml après le traitement par chauffage à sec.

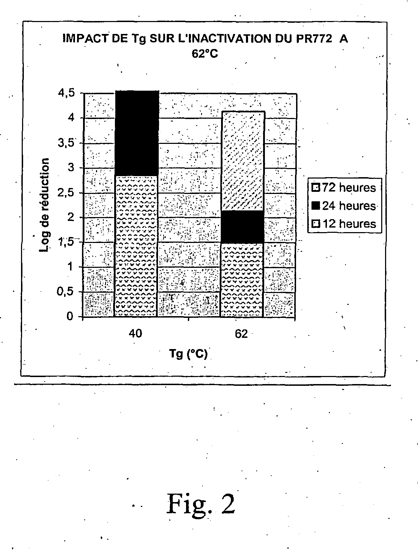

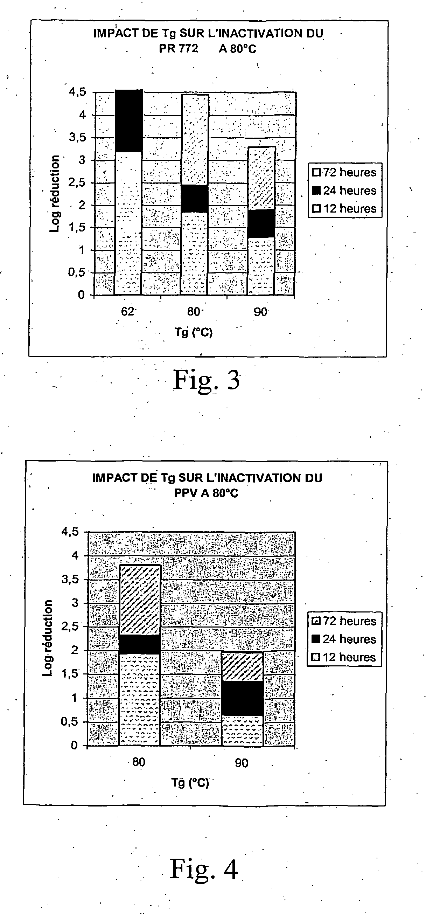

Les résultats sont représentés par les graphiques des figures 2 et 3.
On peut observer que :
- pour un chauffage à T = 80°C,
   1. du produit A dont Tg = 62°C (T-Tg ≈ 20°C), la cinétique d'inactivation est très rapide et le facteur de réduction atteint 4 log₁₀ en moins de 24 heures
   2. du produit C dont Tg = T, le facteur de réduction atteint 4 log₁₀ au bout de 72 heures
   3. du produit D dont Tg = 90°C, le facteur de réduction est inférieur à 4 log₁₀ au bout de 72 heures
- pour un chauffage à 62°C,
   1. du produit A dont Tg = T, le facteur de réduction atteint 4 log₁₀ au bout de 72 heures
   2. du produit B dont Tg = 40°C (T-Tg ≈ 20°C), la cinétique d'inactivation est très rapide et le facteur de réduction atteint 4 log₁₀ en moins de 24 heures

### Exemple 2 : Inactivation du PPV par chauffage à sec dans des lyophilisats :

On mesure le facteur de réduction du PPV à 12, 24 et 72h pour un chauffage à 80°C dans des lyophilisats de Tg = 80 ou 90°C.
Les résultats sont représentés par le graphique de la Figure 4.
On peut observer que pour un chauffage à T = 80°C,
- à Tg = T, le facteur de réduction est proche de 4 log₁₀,
- à T < Tg, le facteur de réduction est faible, de l'ordre de 2 log₁₀.

### Exemple 3 : Inactivation de PPV, HAV, BVDV, PR772 et Phi174 par chauffage à sec à T = Tg dans des lyophilisats :

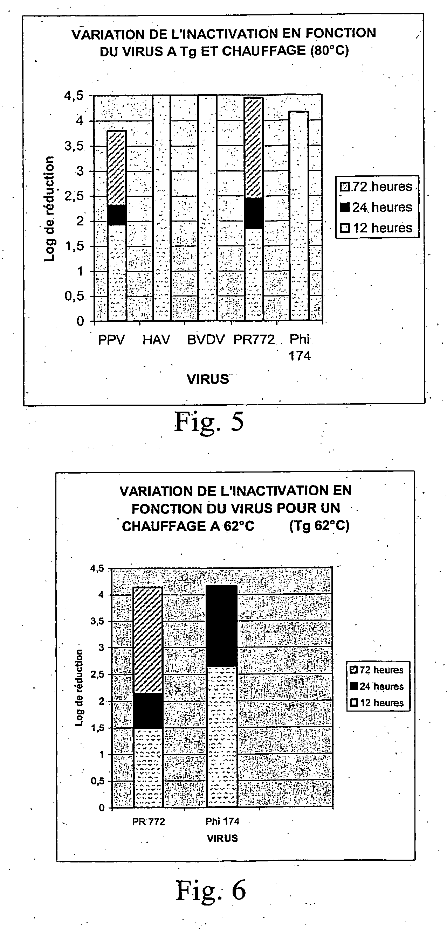

On mesure le facteur de réduction de PPV, HAV, BVDV, PR772 et du bactériophage Phi174 à 12, 24 et 72h pour un chauffage à T = Tg = 80°C (dans un lyohilisat de Tg = 80°C) ou à T = Tg = 62°C (dans un lyophilisat de Tg = 62°C).
Les résultats sont représentés par les graphiques des figures 5 et 6.
On peut observer que, pour des virus peu résistants : HAV, BVDV, Phi174, un chauffage à T = Tg est suffisant pour atteindre un facteur de réduction de 4 log₁₀ dès 24 heures.
Par contre, pour des virus plus résistants : PPV et PR772, le temps de chauffage doit être prolongé à 72 heures pour atteindre un facteur de réduction proche de 4. log₁₀.
Par conséquent, pour ces virus plus résistants, le but étant de les inactiver, on peut favoriser le facteur de réduction virale et la vitesse d'inactivation virale en augmentant la température de chauffage T ou en abaissant la Tg du produit afin d'accroître l'écart entre T et Tg.
Par ailleurs, on préférera soit le domaine T-Tg ≥ 20°C pour favoriser plutôt la vitesse de l'inactivation virale, soit le domaine T-Tg ≤ 20°C pour favoriser plutôt la stabilité du produit:

### Exemple 4 : Impact du chauffage à 80°C, 72h sur les caractéristiques physico-chimiques d'un lyophilisat de FvW en fonction de sa température de transition vitreuse :

Trois lyophilisats de FvW caractérisés par trois températures de transition vitreuse différentes ont été chauffés à 80°C pendant 72 h. Différents paramètres : l'aspect du lyophilisat, son temps de dissolution et l'aspect de la solution ainsi obtenue ont alors été observés.

Les résultats sont présentés dans le Tableau 2.

**Tableau 2 :**

| | | | |
|---|---|---|---|
| % HR | 0,9 | 1,7 | 3,1 |
| Tg (°C) | 74 | 66 | 42 |
| FvW : Rco (UI/ml) | 140 | 120 | 105 |
| Aspect du lyophilisat | normal | Légèrement rétracté | Très rétracté |
| Temps de dissolution (s) | 15 | 35 | 75 |
| Aspect de la solution | limpide | limpide | limpide |

On observe qu'un chauffage à une température T ≥ Tg et T-Tg ≤ 20°C permet de conserver une stabilité satisfaisante du produit bien que la température choisie permette un changement d'état de l'état vitreux vers l'état caoutchouteux.
On observe également qu'un écart trop important entre la température de chauffage et Tg, ici 38°C, est défavorable à la stabilité du produit.
Par conséquent, plus on choisit T proche de Tg, plus on favorise la stabilité du produit..

## Revendications

1. Procédé d'inactivation virale par chauffage à sec d'un virus présent ou potentiellement présent dans un produit biologique séché, **caractérisé par** les étapes suivantes :
a. déterminer la température de transition vitreuse Tg du produit biologique séché à traiter puis,
b. chauffer le produit biologique séché à traiter de l'étape a) à une température de chauffage à sec T supérieure ou égale à la température de transition vitreuse Tg déterminée à l'étape a.

2. Procédé selon la revendication 1, **caractérisé en ce que** la température de transition vitreuse Tg du produit biologique séché est ajustée préalablement à la mise en oeuvre du chauffage à sec.

3. Procédé selon la revendication 1 ou 2, **caractérisé en ce que** le produit biologique séché est un lyophilisat.

4. Procédé selon l'une des revendications 1 à 3, **caractérisé en ce que** le produit biologique séché est une composition contenant une ou plusieurs protéines extraites du plasma sanguin.

5. Procédé selon l'une des revendications 1 à 4, **caractérisé en ce que** la température de chauffage à sec T est choisie pour permettre l'inactivation d'un virus non-enveloppé.

6. Procédé selon l'une des revendications 2 à 5, **caractérisé en ce que** la température de transition vitreuse est augmentée par ajout d'excipients de masse moléculaire élevée au produit biologique ou par diminution de l'humidité du produit biologique.

7. Procédé selon l'une des revendications 2 à 5, **caractérisé en ce que** la température de transition vitreuse est diminuée par ajout de sels ou d'excipients de faible masse moléculaire au produit biologique ou par augmentation de l'humidité du produit biologique.

8. Procédé selon l'une des revendications 1 à 7, **caractérisé en ce que** Tg est mesurée grâce à un thermoanalyseur différentiel à balayage.

9. Procédé selon l'une des revendications 1 à 8, **caractérisé en ce que** T est comprise entre Tg et Tg + 20°C.

10. Procédé selon l'une des revendications 1 à 9, **caractérisé en ce que** la température de chauffage à sec T est choisie pour permettre l'obtention d'un facteur de réduction virale ≥ 3 log₁₀.

11. Procédé selon l'une des revendications 1 à 9, **caractérisé en ce que** la température de chauffage à sec T est choisie pour permettre l'obtention d'un facteur de réduction virale ≥ 4 log₁₀

12. Procédé selon l'une des revendications 9 à 11, **caractérisé en ce qu'**on choisit T de manière à accroître l'écart entre Tg et T dans la limite de Tg +20°C pour favoriser- le facteur de réduction virale et la vitesse d'inactivation virale.

13. Procédé selon l'une des revendications 9 à 11, **caractérisé en ce qu'**on choisit T de manière à diminuer l'écart entre Tg et T pour favoriser la stabilité du produit.

14. Procédé selon l'une des revendications 9 à 13, **caractérisé par le fait que** dans une étape finale, on mesure l'efficacité de l'inactivation virale dans le produit biologique séché traité et, si ladite efficacité est jugée insuffisante, l'inactivation virale du produit biologique séché se poursuit selon l'une des revendications 9 à 11, après accroissement de l'écart entre ladite température de chauffage T et ladite température de transition vitreuse Tg.

15. Procédé selon l'une des revendications 9 à.13, **caractérisé par le fait que** dans une étape finale, on évalue, la stabilité du produit biologique séché traité et, si ladite stabilité est jugée insuffisante, l'inactivation virale du produit biologique séché se poursuit selon l'une des revendications 9 à 11, après une diminution de l'écart entre ladite température de chauffage T et ladite.température de transition vitreuse Tg.

## Claims

1. A viral inactivation method by dry heating of a virus present or potentially present in a dried biological product, **characterized by** the following steps:
a. determining the glassy transition temperature Tg of the dried biological product to be treated and then,
b. heating the dried biological product to be treated from step a) to a dry heating temperature T higher than or equal to the glassy transition temperature Tg as determined in step a.

2. The method according to claim 1, **characterized in that** the glassy transition temperature Tg of the dried biological product is adjusted prior to applying the dry heating.

3. The method according to claim 1 or claim 2, **characterized in that** the dried biological product is a lyophilisate.

4. The method according to any of claims 1 to 3, **characterized in that** the dried biological product is a composition containing one or more proteins extracted from blood plasma.

5. The method according to any of claims 1 to 4, **characterized in that** the dry heating temperature T is selected in order to allow inactivation of a non-enveloped virus.

6. The method according to any of claims 2 to 5, **characterized in that** the glassy transition temperature is increased by adding excipients of high molecular weight to the biological product or by reducing the humidity of the biological product.

7. The method according to any of claims 2 to 5, **characterized in that** the glassy transition temperature is reduced by adding salts or excipients of low molecular weight to the biological product or by increasing the humidity of the biological product.

8. The method according to any of claims 1 to 7, **characterized in that** Tg is measured by a differential scanning thermo-analyzer.

9. The method according to any of claims 1 to 8, **characterized in that** T is comprised between Tg and Tg + 20°C.

10. The method according to any of claims 1 to 9, **characterized in that** the dry heating temperature T is selected so as to obtain a log₁₀ viral reduction factor ≥ 3.

11. The method according to any of claims 1 to 9, **characterized in that** the dry heating temperature T is selected so as to obtain a log₁₀ viral reduction factor ≥ 4.

12. The method according to any of claims 9 to 11, **characterized in that** T is selected so as to increase the difference between Tg and T within the limit of Tg + 20°C in order to promote the viral reduction factor and the viral inactivation rate.

13. The method according to any of claims 9 to 11, **characterized in that** T is selected so as to reduce the difference between Tg and T in order to promote stability of the product.

14. The method according to any of claims 9 to 13, **characterized by** the fact that in a final step, the viral inactivation efficiency in the treated dried biological product is measured and, if said efficiency is assessed to be insufficient, viral inactivation of the dried biological product continues according to any of claims 9 to 11, after increasing the difference between said heating temperature T and said glassy transition temperature Tg.

15. The method according to any of claims 9 to 13, **characterized by** the fact that in a final step, the stability of the treated dried biological product is evaluated, and if said stability is assessed to be insufficient, viral inactivation of the dried biological product continues according to any of claims 9 to 11, after reducing the difference between said heating temperature T and said glassy transition temperature Tg.

## Patentansprüche

1. Verfahren zur Virusinaktivierung durch Trockenerhitzung eines Virus, der in einem getrockneten biologischen Produkt vorhanden oder potentiell vorhanden ist, das durch die folgenden Schritte **gekennzeichnet** ist:
a) Bestimmen der Glasübergangstemperatur Tg des zu behandelnden getrockneten biologischen Produkts, dann
b) Erhitzen des zu behandelnden getrockneten biologischen Produkts aus Schritt a) auf eine Trockenerhitzungstemperatur T größer oder gleich der Glasübergangstemperatur Tg, die in Schritt a) bestimmt wurde.

2. Verfahren nach Anspruch 1, **dadurch gekennzeichnet, dass** die Glasübergangstemperatur Tg des getrockneten biologischen Produkts vor dem Durchführen der Trockenerhitzung angepasst wird.

3. Verfahren nach Anspruch 1 oder 2, **dadurch gekennzeichnet, dass** das getrocknete biologische Produkt ein Lyophilisat ist.

4. Verfahren nach einem der Ansprüche 1 bis 3, **dadurch gekennzeichnet, dass** das getrocknete biologische Produkt eine Zusammensetzung ist, die ein oder mehrere Proteine enthält, die aus Blutplasma extrahiert wurden.

5. Verfahren nach einem der Ansprüche 1 bis 4, **dadurch gekennzeichnet, dass** die Trockenerhitzungstemperatur T ausgewählt ist, um die Inaktivierung eines nicht eingehüllten Virus zu ermöglichen.

6. Verfahren nach einem der Ansprüche 2 bis 5, **dadurch gekennzeichnet, dass** die Glasübergangstemperatur durch Zugabe von Exzipienten mit hoher Molekülmasse zu dem biologischen Produkt oder durch Verringerung der Feuchtigkeit des biologischen Produkts erhöht wird.

7. Verfahren nach einem der Ansprüche 2 bis 5, **dadurch gekennzeichnet, dass** die Glasübergangstemperatur durch Zugabe von Salzen oder Exzipienten mit geringer Molekülmasse zu dem biologischen Produkt oder durch Erhöhung der Feuchtigkeit des biologischen Produkts verringert wird.

8. Verfahren nach einem der Ansprüche 1 bis 7, **dadurch gekennzeichnet, dass** die Tg mit Hilfe eines dynamischen Differenz-Kalorimeters gemessen wird.

9. Verfahren nach einem der Ansprüche 1 bis 8, **dadurch gekennzeichnet, dass** T im Bereich zwischen Tg und Tg +20 °C liegt.

10. Verfahren nach einem der Ansprüche 1 bis 9, **dadurch gekennzeichnet, dass** die Trockenerhitzungstemperatur T ausgewählt ist, um den Erhalt eines virusreduktionsfaktors ≥ 3 log₁₀ zu ermöglichen.

11. Verfahren nach einem der Ansprüche 1 bis 9, **dadurch gekennzeichnet, dass** die Trockenerhitzungstemperatur T ausgewählt ist, um den Erhalt eines Virusreduktionsfaktors ≥ 4 log₁₀ zu ermöglichen.

12. Verfahren nach einem der Ansprüche 9 bis 11, **dadurch gekennzeichnet, dass** T ausgewählt wird, um den Abstand zwischen Tg und T innerhalb der Grenze von Tg +20 °C zu vergrößern, um den Virusreduktionsfaktor und die Geschwindigkeit der Virusinaktivierung zu fördern.

13. Verfahren nach einem der Ansprüche 9 bis 11, **dadurch gekennzeichnet, dass** T ausgewählt wird, um den Abstand zwischen Tg und T zu verringern, um die Stabilität des Produkts zu fördern,

14. Verfahren nach einem der Ansprüche 9 bis 13, **gekennzeichnet durch** die Tatsache, dass in einem abschließenden Schritt die Effizienz der Virusinaktivierung in dem behandelten getrockneten biologischen Produkt gemessen wird, und wenn die Effizienz als unzureichend erachtet wird, die Virusinaktivierung des getrockneten biologischen Produkts nach einem der Ansprüche 9 bis 11 fortgesetzt wird, nachdem der Abstand zwischen der Erhitzungstemperatur T und der Glasübergangstemperatur Tg vergrößert wurde.

15. Verfahren nach einem der Ansprüche 9 bis 13, **gekennzeichnet durch** die Tatsache, dass in einem abschließenden Schritt die Stabilität des behandelten getrockneten biologischen Produkts bewertet wird, und wenn die Stabilität als unzureichend erachtet wird, die Virusinaktivierung des getrockneten biologischen Produkts nach einem der Ansprüche 9 bis 11 fortgesetzt wird, nachdem der Abstand zwischen der Erhitzungstemperatur T und der Glasübergangstemperatur Tg verringert wurde.
